# EUROPEAN PATENT SPECIFICATION

(11) **EP 3 747 473 B1**
(45) Date of publication and mention of the grant of the patent: **21.09.2022**
(21) Application number: 18903486.1
(22) Date of filing: 31.01.2018
(51) Int. Cl.: A61L 17/06, A61L 17/00, A61L 17/04, A61L 17/08, A61L 17/10, A61L 17/12

(54) **COMPOSITE SURGICAL SUTURE MATERIAL BASED ON POLYFILAMENTOUS TITANIUM THREAD AND BIORESORBABLE POLYMERS**
CHIRURGISCHES VERBUNDNAHTMATERIAL AUF DER BASIS VON POLYFILAMENTÄREM TITANFADEN UND BIORESORBIERBAREN POLYMEREN
MATÉRIAU CHIRURGICAL COMPOSITE POUR SUTURES À BASE D'UN FIL DE TITANE À FILAMENTS MULTIPLES ET DE POLYMÈRES BIORÉSORBABLES

(43) Date of publication of application: 09.12.2020
(73) Proprietor: Titanium Textiles AG, 18182 Rostock-Bentwisch (DE)
(72) Inventor: KAZANTSEV, Anton Anatolevich, Ekaterinburg, 620062 (RU); YUSUPOV, Ajrat Auhatovich, Pyshma, 624090 (RU)
(74) Representative: Hoffmann Eitle
(86) International application number: PCT/RU2018/000050
(87) International publication number: WO 2019/151887

(56) References cited:
- WO-A2-2009/059147
- WO-A2-2009/132284
- RU-U1- 116 035
- RU-U1- 173 623
- FEDOROV P.G. et al.: Sovremennye shovnye materialy (obzor literatury) Acta Biomedica Scientifica, vol. 2, no. 6, 2017, pages 157-162, XP055726012,

## Description

### FIELD OF THE INVENTION

The invention relates to the field of medicine and medical technology and is aimed at improving the technical properties of the surgical suture material.

### STATE OF ART

A surgical suture material in the form of a thread made of an alloy based on titanium nickelide is known from the prior art (RU 2234868 C2, publ. 27.08.2004).

Disadvantages of this solution are that the material is made of titanium nickelide, does not have a smooth surface, it has a so-called "sawing effect" on tissues and cannot serve as a depot of drugs. In addition, the phase heterogeneity of titanium nickelide creates likelihood of the diffusion of nickel ions into the surrounding tissues, which can be a source of persistent inflammation.

From the prior art, a solution is known that is the closest to the claimed one (RU 116035 U1, publ. 20.05.2012). Surgical titanium suture material (options)). The surgical suture material is made of a titanium alloy; it comprises a needle tip interconnected with a multifilament suture thread, wherein the monothreads of the polyfilament suture thread intersect with each other at an angle to the longitudinal axis of the suture material.

Disadvantages of this solution are that the material is made of conventional polyfilament titanium material, does not have a smooth surface, it has a so-called "sawing effect" on tissues and cannot serve as a depot of drugs, and also there is a high risk of suture material breakage. In addition, most polymers have poor adhesion to the existing titanium suture material and do not allow the formation of self-fixing elements in the form of grips, thorns, petals, etc. designs thereon.

RU 173623 U1, (publ. 04.09.2017) discloses a suture comprising a polymer matrix coating/ embedding Ti filaments.

The claimed suture material makes it possible to substantially overcome said disadvantages inherent in the prototype.

### DISCLOSURE OF THE INVENTION

The technical problem solved by the proposed technical solution is the development of a suture material having high strength, elasticity and plasticity, devoid of a "sawing" effect on tissues, with a low coefficient of sliding friction, and also capable of carrying antibacterial and other medicinal substances in its structure, wherein the material should be designed for a long-term presence in the body and possessing a high biological inertness in the late postoperative period, not initiate or promote aseptic inflammation associated with the presence thereof in the body.

The technical result consists in an increase in the strength, elasticity and plasticity of the suture material, the absence of a "sawing" effect on tissues, an increase in the sliding friction coefficient, and also in providing the opportunity to serve as a depot for antibacterial and other medicinal substances, in ensuring a long presence in the body and possessing a high biological inertness in the late postoperative period, excluding the likelihood of initiation and persistence of aseptic inflammation associated with the presence of the material in the body.

The technical result is achieved due to the fact that the composite surgical suture material consists of titanium monothreads, combined in a polyfilament thread, wherein a bioresorbable polymer material is present in the gaps between the monothreads and on their surface, and wherein the monofilaments have a relief surface.

The monothreads are made of GRADE-5 titanium alloy.

The relief surface of the monothread is made by chemical etching.

The bioresorbable polymer material is a polymer from the group of polylactic acid, silk fibroin, and polyhydroxybutyrate.

The polyfilament thread is made with a diameter of 0.04 mm to 2 mm.

The polyfilament thread is made in the form of a tape 0.2-1 mm thick and 3-5 mm wide.

The structure of the bioresorbable polymer material contains at least one drug.

Self-fixing elements are formed on the surface of the material in the form of grips, thorns having a triangular shape with an acute angle faced in the direction of movement of the material, and the opposite side of the triangle faced against the direction of movement.

Self-fixing elements are formed on the surface of the material in the form of petals folded in the same direction, having sharp tips faced against the direction of movement of the surgical material.

### BRIEF DESCRIPTION OF DRAWINGS

F
FIG. 1. Image of a composite surgical suture material, twisted in the form of a cable, wherein a bioresorbable polymer is present in the gaps between the threads and on the surface.
FIG. 2. Image of a composite surgical suture material, woven in the form of a microtube, wherein a bioresorbable polymer is present in the gaps between the threads and inside.
FIG. 3. Image of a composite surgical suture material in the form of a tape, wherein a bioresorbable polymer is present in the gaps between the threads and on the surface.
FIG. 4a. Schematic representation of a cross-section of the threads without relief.
FIG. 4b. Schematic representation of a cross-section of the threads with a relief.
FIG. 5a. Schematic representation of the threads without relief.
FIG. 5b. Schematic representation of the threads with a relief.
FIG. 6a. Schematic representation of a cross-section of the thread with sharp-pointed defects.
FIG. 6b. Schematic representation of a cross-section of the thread with smoothed defects.
FIG. 7. Image of a composite surgical suture material with self-fixing elements in the form of petals, formed from a bioresorbable polymer.
FIG. 8. Image of a composite surgical suture material with self-fixing elements in the form of grips, thorns, formed from a bioresorbable polymer.

### IMPLEMENTATION OF THE INVENTION

The suture material is intended for use in surgical interventions in various areas of surgery (abdominal, dentistry, orthopedics, including for cerclage suturing). It is used for suturing of muscle-fascial formations, suturing of the capsule of organs, suturing of tendons, for bone grafting, for fixing implants, etc.

The basis of the invention lies in the combination of a polyfilament titanium thread having optimal strength and elasticity for surgery and biologically resorbable polymers that exclude the sawing effect.

Composite surgical suture material can consist of 3-96 titanium monothreads 2, combined in a polyfilament thread 1, wherein a bioresorbable polymer material 3 is present in the gaps between the monothreads and on their surface. This material can be polymers from the group of polylactic acid, silk fibroin, polyhydroxybutyrate, etc.

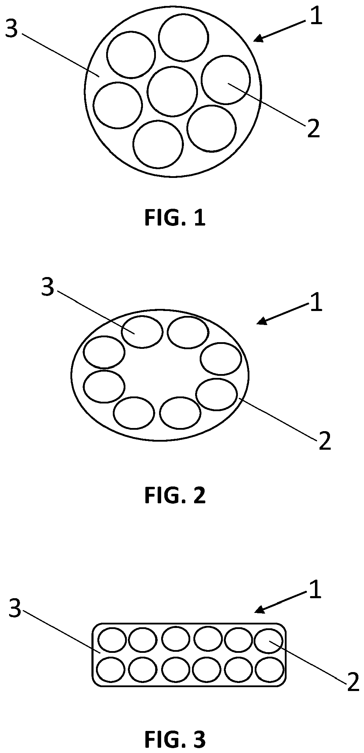

The polyfilament thread 1 can be twisted in the form of a cable (FIG. 1), braided in the form of a microtube (FIG. 2), or knitted in another way.

The suture material (polyfilament thread 1) can have a diameter of 0.04 mm to 2 mm, and also can have the form of a tape 0.2-1 mm thick and 3-5 mm wide. (FIG. 3)

Titanium monothreads can be made of GRADE-1 (VT1.00, VT1.00 wa) or GRADE-5 (VT6) titanium and have a relief surface.

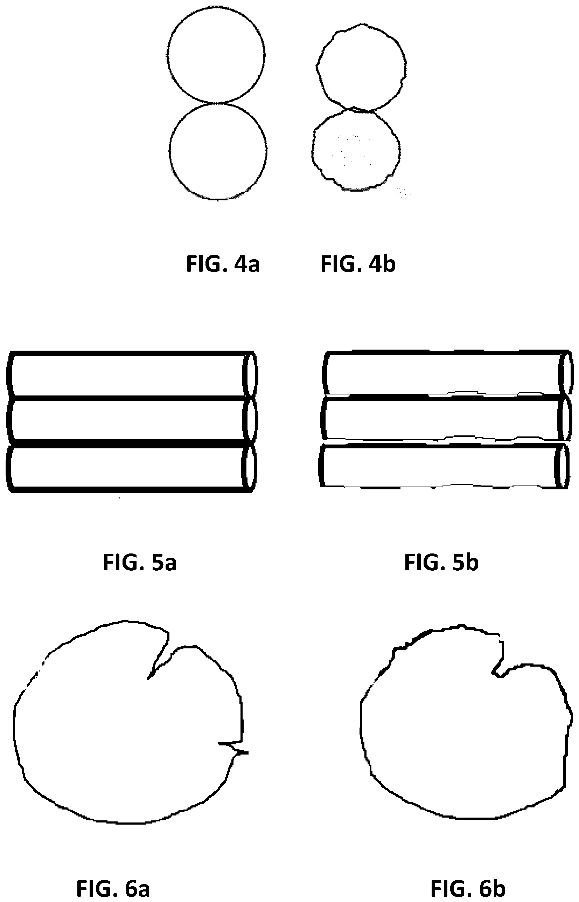

The polyfilament relief titanium thread is characterized by a decrease in the area of the wire contacts of individual monothreads, which is due to a decrease in their diameter, achieved, for example, using chemical etching yet at the stage of formation of the polyfilament material (FIG. 4b), in comparison with the starting material (FIG. 4a).

Figures 4a and 4b show a cross-section of the original and relief titanium threads. They show that a decrease in the contact area results from both a decrease in the diameter and change in the surface structure, which takes the form of a chaotically formed relief. At the same time, when forming the relief, slotted gaps appear between the threads, thus, the relief polyfilament titanium thread has an increased porosity. The result is a decrease in the length of contact and the contact area of the threads used.

Figures 5a and 5b show that the length of contact of the threads, and therefore the contact area in FIG. 5a is much larger than in FIG. 5b due to the presence of slotted gaps.

The surface relief is a significant factor in improving the fixation of the bioresorbable polymer to the material, since the surface roughness increases the sliding friction coefficient. Due to the penetration of the polymer into the slotted gaps, the adhesion of the polymer to the titanium thread is increased.

Technological processes that make it possible to obtain relief titanium thread are: chemical etching, electrochemical polishing, etc. Such treatment, which reduces the diameter of the titanium thread by 5-35% of the initial diameter, makes it possible to significantly reduce the braiding density of the material and, at the same time, achieve maximum plasticity. Thanks to these processes, a specific surface relief appears on the surface of the material: chaotically located depressions and bumps with a rounded surface

Distinctive feature of the surgical material is the unevenness of the titanium thread diameter (FIG. 4b). Fluctuation of the thread diameter of the monofilament thread (depending on the initial diameter) is 0.1-10 µm (FIG. 4b), which is caused by the impossibility of ensuring the uniformity of the effect of the reagents on the monofilament threads comprised in the structure of the suture material and having a different surface area outside of contact with other threads.

In addition, in the process of obtaining a relief on the surface of the thread, longitudinal sharp-pointed defects (FIG. 6a) arising from drawing of the thread are smoothed; the smoothing of defects after treatment is shown in FIG. 6b. Smoothing of longitudinal defects, which are the concentrators of internal stress, equalize the residual stress in the thread itself and reduces the risk of suture material breakage.

In addition, when a relief is obtained on the surface of the thread by chemical etching, the surface layers are cleaned from the remnants of technological lubricant and other impurities, which significantly improves the biological properties of the material.

The high plasticity of the relief thread minimizes the spring properties, reduces the likelihood of biomechanical conflict between the suture material and body tissues and makes it possible to place the material in delicate anatomical structures without the risk of injury. Composite suture material made of the relief titanium thread is freely expanded in the area of the operating wound, does not form unnecessary loops, and easily forms friction knots.

High porosity increases the rate of penetration of biological fluids into the suture material during the biodegradation of the polymer, accelerates the process of its colonization by fibroblasts and osteoblasts, and improves biological integration.

After biodegradation, the surface relief of the thread significantly improves the fixation of fibrin fibers thereon, thereby facilitating the attraction of fibroblasts serving as a source of newly formed connective tissue.

The material can be completed with one or two nontraumatic needle tips.

In some cases, the composite suture material can be used as a self-fixing material in clinical situations where it is desirable to avoid bending of friction knots.

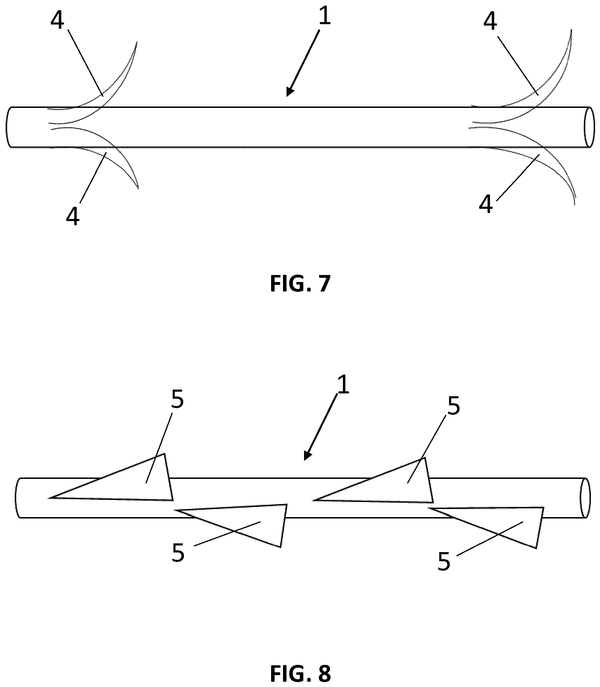

Composite suture material can contain self-fixing elements, which are petals 4 (FIG. 7), 1-4 mm long and ¼-1 diameter of the suture material wide, folded in the same direction and having sharp tips faced against the direction of movement of the surgical material, or grips, thorns 5 (FIG. 8) with a height of 0.1-2 mm, having a triangular shape with an acute angle faced in the direction of movement of the material, and the opposite side of the triangle faced against the direction of movement, so as to prevent the counter movement of the surgical suture material. The distance between the self-fixing elements along the length can be from 0.5 to 2.5 cm. The self-fixing elements are also made of polymer material.

The incorporation of polymers makes it possible to significantly improve the ergonomics of the material, eliminate the "sawing effect" and control biological processes in the operating room in the short term, while the biological inertness of titanium allows a full-fledged postoperative scar to form in the long term.

The incorporation of polymer materials into the structure of the polyfilament titanium thread leads to physical adhesion of such polymer materials to the thread over the entire surface and in the gaps between the monothreads (monofilament threads). As a result of such physical contact, an even distribution of the load between the individual monothreads is achieved, avoiding overstressing of any one or a group of monothreads during operation and thus further enhancing the strength of the suture material.

In addition, the polymer structures can contain at least one drug: antibiotics, antiinflammatory drugs, etc. Being gradually released, drugs can have a prolonged therapeutic effect, preventing inflammation and the development of wound infection.

The incorporation of polymers opens the technological potential of development of self-fixing elements on a composite suture material, which possibility is not available when using a conventional titanium thread. At the same time, the formed self-fixing elements are much stronger than the existing notches on polymer materials.

The claimed solution allows:
To create high strength and elasticity due to the presence of the polyfilament titanium threads.

To smooth out irregularities arising in the process of production of the polyfilament titanium material.

To strength the material even more, providing an even distribution of the tension load between all monofilament threads at the same time.

To strength the adhesion of the bioresorbable polymer to the surface of the titanium thread.

To strength the adhesion of the bioresorbable polymer to the titanium thread in a structural manner, due to the penetration of the polymer into the slot gaps.

To provide the possibility of temporary deposition of drugs with gradual release from the suture material.

The resulting material has a smooth surface and eliminates the "sawing effect", which compares favorably with conventional polyfilament titanium material, for which obtaining a smooth surface is an unsolvable task.

To obtain self-fixing elements on the surface of the suture material

To minimize the likelihood of persistent aseptic inflammation in the late postoperative period.

After a relatively short period of time, the polymer is resorbed by cells of the body, and an inert titanium thread remains in place, which does not harm the body.

### Example 1

A model of a ventral hernia on the outer side of the anterior abdominal wall was obtained in three laboratory animals (rabbits, 4 months) through an open-cut surgery, and a 3x3 cm mesh implant was installed. The implant was placed retroperitoneally. The subcutaneous fat was sutured with the suture material consisting of three monofilament threads with a diameter of 60 µm and polylactic acid with self-fixing elements in the form of grips, thorns. When working with suture material, it was noted good slippage in the tissues with simultaneous self-fixation (no counter movement). The skin was sutured with interrupted sutures. Postoperative wound healing by primary intention. After 3 months, the animals were withdrawn from the experiment. When studying morphologic changes: a whitish scar was found over the entire surface of the mesh implant. In the area of suture material application, traces of polylactic acid were not detected; upon microscopic examination, the structure of the postoperative scar was represented by ordered connective tissue fibers without signs of aseptic inflammation.

### Example 2

Interventions for implantation a composite suture material were performed in rats under general anesthesia with intraperitoneal Nembutal 30 mg/kg. The animals were 6 months old at the moment of the intervention.

An operating wound was formed within the lumbar region of a laboratory animal by dissecting the skin, muscles and subcutaneous fatty tissue in the lumbar region. The wound was contaminated with a culture of *Staphylococcus aureus* 10⁹ CFU/ml. All animals were divided into three groups of 6 individuals each.

Both internal and skin sutures were applied. For suturing the wound in the first group of six individuals (control), a polyfilamentmultifilament suture material made of polypropylene thread (most often used in surgery) was used; for suturing the wound in the second group, in the first three individuals the claimed composite suture material was used, with a thread diameter of 0.04 mm, containing in addition to titanium threads 35% (vol/vol) of polylactic acid and 3% of vancomycin; in the second group (three individuals), the claimed composite suture material was used, with a thread diameter of 2 mm, containing in addition to titanium threads 35% (vol/vol) of polyhydroxybutyrate and 3% of vancomycin; and for suturing the wound in the third group (three individuals), the claimed composite suture material in the form of a tape 0.2 mm thick and 3 mm wide was also used, containing in addition to titanium threads 35% (vol/vol) of polylactic acid and 5% of tobramycin.

The observation period lasted for 7 days.

It was noted that in the second and third groups of animals, the inflammation was localized, the biological inertness of titanium allowed a full-fledged postoperative scar to be formed, which scar had good elasticity and high strength, no sawing effect was observed during working with suture material, dense overgrowth of connective tissue fibers on the polyfilament thread provided a reliable fixation between the tissue and the suture material, microscopic gaps between the suture material and the connective tissue were not detected. In the control group of laboratory animals, breakage was observed and a sawing effect was exerted in the process of working with suture material, delaminating, bends and twisting of individual monofilaments into loops causing breakage thereof were observed. During healing, infiltrative necrotic changes in the tissues of the surgical wound were present. The most pronounced changes were observed on the 7th day of the study. Differences between the study groups and the control series were significant throughout the whole observation period. Thus, the efficiency of using the claimed composite suture material was noted.

The claimed composite surgical suture material based on polyfilament titanium thread and bioresorbable polymers has increased strength, elasticity and plasticity, ensuring the absence of a "sawing" effect on tissues, and is also capable of carrying antibacterial and other medicinal substances in its structure, preventing the development of infectious inflammation in the wound. In the late postoperative period, after polymer resorption, the claimed suture material has a high biological inertness, excluding the initiation and persistence of aseptic inflammation associated with the presence of a foreign body in the body.

## Claims

1. Composite surgical suture material consisting of titanium monothreads (2) combined in a polyfilament thread (1), wherein a bioresorbable polymer material (3) is present in the gaps between the monothreads (2) and on their surface, and wherein the monothreads (2) have a relief surface.

2. Composite surgical suture material according to claim 1, wherein the monothreads (2) are made of GRADE-5 titanium alloy.

3. Composite surgical suture material according to claim 1, wherein the relief surface of the monothread (2) is obtained by chemical etching.

4. Composite surgical suture material according to claim 1, wherein the bioresorbable polymer material (3) is a polymer from the group of polylactic acid, silk fibroin, and polyhydroxybutyrate.

5. Composite surgical suture material according to claim 1, wherein the polyfilament thread (1) is made with a diameter of 0.04 mm to 2 mm.

6. Composite surgical suture material according to claim 1, wherein the polyfilament thread (1) is made in the form of a tape 0.2-1 mm thick and 3-5 mm wide.

7. Composite surgical suture material according to claim 1, wherein the structure of the bioresorbable polymer material (3) contains at least one antibacterial drug.

8. Composite surgical suture material according to claim 1, wherein self-fixing elements are formed on the surface of the material in the form of grips, thorns having a triangular shape with an acute angle faced in the direction of movement of the material, and the opposite side of the triangle faced against the direction of movement.

9. Composite surgical suture material according to claim 1, wherein self-fixing elements are formed on the surface of the material in the form of petals folded in the same direction, having sharp tips faced against the direction of movement of the surgical material.

## Patentansprüche

1. Chirurgisches Verbundnahtmaterial, bestehend aus Titanmonofäden (2), die in einem Polyfilamentfaden (1) zusammengefasst sind, wobei ein bioresorbierbares Polymermaterial (3) in Lücken zwischen den Monofäden (2) sowie auf deren Oberfläche vorliegt und wobei die Monofäden (2) eine reliefartige Oberfläche aufweisen.

2. Chirurgisches Verbundnahtmaterial gemäß Anspruch 1, wobei die Monofäden (2) aus GRADE-5-Titanlegierung hergestellt sind.

3. Chirurgisches Verbundnahtmaterial gemäß Anspruch 1, wobei die reliefartige Oberfläche der Monofäden (2) mittels chemischer Ätzung erhalten ist.

4. Chirurgisches Verbundnahtmaterial gemäß Anspruch 1, wobei das bioresorbierbare Polymermaterial (3) ein Polymer aus der Gruppe von Polymilchsäure, Seidenfibroin und Polyhydroxybutyrat ist.

5. Chirurgisches Verbundnahtmaterial gemäß Anspruch 1, wobei der Polyfilamentfaden (1) mit einen Durchmesser von 0,04 mm bis 2 mm ausgeführt ist.

6. Chirurgisches Verbundnahtmaterial gemäß Anspruch 1, wobei der Polyfilamentfaden (1) in Form eines Bandes mit einer Dicke von 0,2-1 mm und einer Breite von 3-5 mm ausgeführt ist.

7. Chirurgisches Verbundnahtmaterial gemäß Anspruch 1, wobei die Struktur des bioresorbierbaren Polymermaterials (3) mindestens ein antibakterielles Mittel enthält.

8. Chirurgisches Verbundnahtmaterial gemäß Anspruch 1, wobei Selbstfixierelemente auf der Oberfläche des Materials in Form dreieckiger Dorne vorgesehen sind, die einen spitzen Winkel in der Bewegungsrichtung und eine jeweils entgegengesetzte Seite des Dreiecks entgegen der Bewegungsrichtung aufweisen.

9. Chirurgisches Verbundnahtmaterial gemäß Anspruch 1, wobei Selbstfixierelemente auf der Oberfläche des Materials in Blattform vorgesehen sind, die in gleicher Richtung ausgerichtet sind und scharfe Spitzen entgegen der Bewegungsrichtung des chirurgischen Nahtmaterials aufweisen.

## Revendications

1. Matériau de fil de suture chirurgical composite consistant en des monofils de titane (2) combinés en un fil polyfilament (1), dans lequel un matériau polymère biorésorbable (3) est présent dans les espaces entre les monofils (2) et sur leur surface, et dans lequel les monofils (2) présentent une surface en relief.

2. Matériau de fil de suture chirurgical composite selon la revendication 1, dans lequel les monofils (2) sont en alliage de titane GRADE 5.

3. Matériau de fil de suture chirurgical composite selon la revendication 1, dans lequel la surface en relief du monofil (2) est obtenue par attaque chimique.

4. Matériau de fil de suture chirurgical composite selon la revendication 1, dans lequel le matériau polymère biorésorbable (3) est un polymère appartenant au groupe de l'acide lactique, de la fibroïne, et du polyhydroxybutyrate.

5. Matériau de fil de suture chirurgical composite selon la revendication 1, dans lequel le fil polyfilament (1) est fabriqué avec un diamètre de 0,04 mm à 2 mm.

6. Matériau de fil de suture chirurgical composite selon la revendication 1, dans lequel le fil polyfilament (1) est fabriqué sous forme d'un ruban de 0,2-1 mm d'épaisseur de 3-5 mm de large.

7. Matériau de fil de suture chirurgical composite selon la revendication 1, dans lequel la structure du matériau polymère biorésorbable (3) contient au moins un médicament antibactérien.

8. Matériau de fil de suture chirurgical composite selon la revendication 1, dans lequel des éléments autofixants sont formés sur la surface du matériau sous forme de parties de préhension, des épines présentant une forme triangulaire avec un angle aigu orienté dans la direction de mouvement du matériau, et le côté opposé du triangle faisant face à la direction de mouvement.

9. Matériau de fil de suture chirurgical composite selon la revendication 1, dans lequel les éléments autofixants sont formés sur la surface du matériau sous forme de pétales pliés dans la même direction, présentant des pointes acérées faisant face à la direction de mouvement du matériau chirurgical.
